Europäisches Patentamt

(19) European Patent Office

Office européen des brevets

(11) Publication number: **0 059 739**
**B1**

(12) **EUROPEAN PATENT SPECIFICATION**

(45) Date of publication of patent specification: **31.07.85**

(21) Application number: **81902569.3**

(22) Date of filing: **15.09.81**

(86) International application number:
**PCT/US81/01234**

(87) International publication number:
**WO 82/00953 01.04.82 Gazette 82/09**

(51) Int. Cl.⁴: **B 32 B 27/08, A 61 F 5/44,**
**C 08 L 23/28, C 08 L 23/08,**
**B 32 B 7/02**

(54) COMPOSITE FILM FORMED FROM A THERMOPLASTIC POLYMERIC RESINOUS BLEND.

(30) Priority: **16.09.80 US 187660**

(43) Date of publication of application:
**15.09.82 Bulletin 82/37**

(45) Publication of the grant of the patent:
**31.07.85 Bulletin 85/31**

(84) Designated Contracting States:
**AT CH DE FR GB LI NL SE**

(56) References cited:
**US-A-3 086 957**
**US-A-3 302 647**
**US-A-3 355 519**
**US-A-3 396 901**
**US-A-3 409 706**
**US-A-3 524 795**
**US-A-3 551 526**
**US-A-3 575 779**
**US-A-3 887 648**
**US-A-3 957 910**

(73) Proprietor: **THE DOW CHEMICAL COMPANY**
**Dow Center 2030 Abbott Road Post Office Box 1967**
**Midland Michigan 48640 (US)**

(72) Inventor: **TUSIM, Martin Howard**
**1495 Londondale Parkway**
**Newark, OH 43055 (US)**

(74) Representative: **Hann, Michael, Dr. et al**
**Patentanwälte Dr. Michael Hann Dr. H.-G. Sternagel Marburger Strasse 38**
**D-6300 Giessen (DE)**

Courier Press, Leamington Spa, England.

**0 059 739**

## Description

This invention relates to a thermoplastic multilayer composite film in which at least one layer comprises a blend of chlorinated polyolefin and an olefinic polymer or copolymer and at least a layer of another material, said other layer being preferably a moisture and odor impermeable barrier layer. In more specific aspects the invention relates to wrapping films and in particular to medical pouches such as ostomy bags, packaging containers and protective clothing.

Chlorinated polyolefins are well known and have been used as fire-retardant additives for polyolefins. US—A—3 086 957 describes a composition comprising chlorinated polyethylene with a chlorine content of from 10 to 70 percent by weight, polyethylene (5 to 50 percent by weight of the composition) and a stabilizer (3 percent by weight of the composition). The single specific Example discloses a chlorinated polyethylene with a chlorine content of 24 percent by weight. The patent does not refer to the preparation of films and does not give any teaching of the properties of films made from the disclosed compositions.

Japanese Patent Publication No. 9 010/1967 refers to a polyethylene composition which has been rendered flame-retardant by blending polyethylene with chlorinated polyethylene, antimony oxides and a stabilizer. This publication does also not disclose or teach the processing of the flame-retardant composition into films.

In this invention the preferred application of the multi-layered composite film is as an ostomy bag where there has existed a great need for a "quiet" or "rustle-free" bag which could be worn by its user in greater comfort and with a substantially lesser degree of awareness of the bags presence next to the wearer's skin due to its noiseless characteristics. Surgical drainage pouches, sometimes known as ostomy bags, permit drainage from parts of the body and are temporarily worn by a patient such as that illustrated in U.S. Patent No. 3,302,647, for example. Thus, an ostomy bag should have minimal or no "rustle", i.e., noise, when worn because the resulting noise can often be embarrassing or disturbing to the wearer. Also, without such noise, it is easier for the wearer to remove from his mind the fact that he is wearing an ostomy bag. Additionally, prior containers including ostomy bags, have been unable to provide adequate odor and moisture barrier properties while at the same time achiving a desirable structural thinness and a high degree of quietness.

Present films for containers of this nature generally are made from polyethylene films or from more expensive plasticized polyvinyl chloride film, plasticized polyvinylidene chloride copolymer films and multilayered structures such as ethylene vinyl acetate/polyvinylidene chloride/ethylene vinyl acetate combination films. To a greater or lesser extent, the more desirable thin gauge containers having a high degree of odor and vapor barrier characteristics also have a high noise level such that they "rustle" when worn by the user. Accordingly, there is a need for a substantially noise-free product which is economical to make, has good odor and moisture barrier properties, and has sufficient strength for its intended purposes.

The purpose of the present invention is to obtain a practical thermoplastic film particularly one useable in medical applications, which has physical properties which provide a high degree of quietness. Such a product should also have sufficient softness and comfort, heat sealability, strength or toughness, e.g., puncture resistance and, where desirable or necessary, adequate vapor, i.e., moisture, gas, and odor barrier properties.

The present invention provides a thermoplastic multilayer composite film having improved dart impact strength comprising at least one layer (a) formed from a composition of a blend of polymeric resins wherein a first component of said blend comprises from 40 to 95 weight percent of a chlorinated poly olefin having from 25 to 50 weight percent chemically combined chlorine, and wherein a second component of the blend comprises from 2 to 57 weight percent of at least one olefinic polymer or copolymer, and from 1 to 3 weight percent of a stabilizing composition to prevent degradation of the chlorinated poly olefin at extrusion temperatures for forming the film, and at least one layer (b) formed from another polymeric material adhered to at least one surface of said layer (a).

Such a composite film has all the desired properties as described above. Where the film is used as an ostomy bag it must possess a degree of quietness which is near rustle-free and insignificant to the background noise to accomplish its desired purpose. Preferably the composite film according to the invention has a noise level of less than 4000 Hertz when measured against a background noise of about 600 Hertz.

In a preferred embodiment of the invention the layer (a) of the composite film comprises from 55 to 80 weight percent chlorinated polyethylene having from 30 to 42 weight percent chemically combined chlorine and the second component comprises from 17 to 42 weight percent of at lesat one olefinic polymer or copolymer. The second component of the blend for layer (a) of the composite film according to the invention is preferably a low, linear low, or medium density polyethylene or a copolymer of ethylene and at least one ethylenically unsaturated comonomer.

The preferred thickness of layer (a) of the film according to the invention is from 63 to 152 µm. The overall thickness of a multilayer film is from 25.4 to 254 µm and preferably from 63 to 152 µm.

In a particular three layered composite film structure for use as ostomy pouches, the outer or skin layers are each about 33 µm in thickness and the barrier resin core layer is about 6 µm providing for a composite thickness of about 72 µm of course, thicknesses of the individual film layers can vary widely depending on the degree of toughness and barrier properties desired for a particular application. Multilayer

2

**0 059 739**

film structures used in this invention can be made by coextrusion methods and apparatuses such as typified by U.S. Patent Nos. 3,354,506, 3,557,265 and 3,625,348.

In a preferred embodiment of the invention the composite film comprises in the blend of layer (a) as stabilizing composition 1 to 2 percent by weight calcium stearate and 1 percent by weight of epoxidized soybean oil.

According to another preferred embodiment of the invention the composite film comprises as layer (b) a vapor barrier layer which is preferably a vinylidene-vinyl chloride copolymer. Preferably a layer (a) is adhered to opposite sides of the layer (b). The composite film according to the invention can also include at least one synthetic resinous glue layer positioned between layer (a) and layer (b). The multilayer composite film can further include at least one synthetic resinous glue layer positioned on at least one surface of layer (a) or on layer (b). In one particular embodiment of the invention the composite film is laminated or coextruded onto a substrate.

The invention further resides in a medical pouch comprising a multilayered composite film according to the invention.

Figure 1 is an approximate graphical representation of the intensity and frequency levels of background noise against which the noise levels of prior art and other products, and the products of this invention were measured;

Figures 2 and 3 are approximate graphical representations of the intensity and frequency levels of prior art and other products, respectively; and

Figure 4 is an approximate graphical representation of the intensity and frequency level of a typical product made in accordance with the principles of this invention.

The invention includes an improvement over current ostomy bag film, such as typified in U.S. Patent No. 3,524,795. In this particular prior art structure, polyethylene skins are formed about a Saran® (polyvinylidene chloride copolymer) resin core with glue layers between the skin and core to adhere the skins to the core. Such a film exhibits a noise level which, if it could be reduced by 15 or 20 percent, would make the film practically rustle-free and thus very attractive to users. It has now been discovered that by introducing a blend of resins, a first component of which is chlorinated polyethylene, and the second component of which is a low-density polyethylene, that the desired degree of quietness in the film could be achieved. It was additionally discovered that not only was quietness assured, but that the glue layers between the skins and the core while permissible for some applications, would not usually be required because of the natural adhesive character of the chlorinated polyethylene resin. One preferred embodiment of the invention employs the use of a chlorinated polyethylene resin such as that disclosed in an article entitled, "Structure-Property Relationships of Chlorinated Polyethylene", by N. K. Kalfoglou and H. L. Williams in *Polymer Engineering and Science*, May, 1972, Volume 12, No. 3, starting at page 224 and in U.S. Patent Nos. 3,396,901, 3,454,544 and 3,678,873.

More specifically, the chlorinated olefin polymers found to be useful for the purposes of the present invention are preferably prepared by the chlorination, in suspension in an inert diluent, of polyethylene or interpolymers containing at lesat about 90 mole percent of ethylene in the polymer molecule with any remainder being one or more ethylenically unsaturated comonomers wherein such polymers are preferably of an essentially linear structure. Chlorinated olefin polymers contain from 25 to 50, and preferably from 30 to 42 weight percent of chemically combined chlorine and are characterized by having a relative crystallinity of between 15 and 28 percent when containing about 25 weight percent chlorine and a relative crystallinity of less than about 10 percent when containing about 34 or more weight percent chlorine, wherein said relative crystallinity is a measure of the ratio of the crystalline peak areas to the sum of the amorphous plus crystalline peak area as determined by conventional X-ray diffraction techniques.

Exemplary of preferred polyolefin materials to be chlorinated are those distinct species and varieties of essentially linear and unbranched highly porous, finely divided polymers containing at least about 90 mole percent ethylene in the polymer molecule with the remainder being one or more ethylenically unsaturated comonomers such as the nonaromatic hydrocarbon olefins having 3 or more carbon atoms, including propylene, butene-1 and butene-2 and 1,7-octadiene; cycloaliphatic olefins such as 1,5-cyclopentene and cyclooctadiene, substituted olefins such as acrylic acid and its esters; conjugated diolefins such as butadiene; and the alkenyl aromatic compounds such as styrene and its derivatives.

The olefinic polymer resins used as the secondary component of the blend in the present invention include low, linear low, or medium density polyethylene or ethylene copolymers which comprise ethylene with at least one monoethylenically unsaturated comonomer, especially another lower olefin or a carboxylic acid or an alkyl ester of a monoethylenically unsaturated carboxylic acid. Examples of such copolymers include ethylene-propylene, ethylene acrylic acid, ethylene methyl methacrylate and ethylene vinyl acetate. Blends of one or more of the ethylenic polymer resins may also be used as the secondary component. Methods of making ethylenic polymer resins described hereinabove are readily known in the art.

The term "low-density polyethylene" as used herein means branched polyethylene having a density from 0.910 to 0.930 g/cc and a melt index from 0.1 to 10 dg/min. The term "linear low-density polyethylene"

---

® Registered Trademark

as used herein means low density polyethylene made by the low pressure polyethylene process. "Medium-density polyethylene" may have a density from 0.931 to 0.940.

A chlorinated polyethylene (CPE) resin having a chlorine content range of from 25 to 50 weight percent results in an improved overall processability of the blend and in improved properties of the resulting film. The film quietness exhibited by the chlorinated polyethylene blend may be related to its rubbery or elastic nature. A preferred CPE resin has a chlorine content of about 36 weight percent, a melt index of about 2 and a density of about 1.16 with a crystallinity of less than about 2 percent.

A preferred resin blend for layer (a) of the film of this invention can be, for example, one having from 70 to 80 percent by weight of the chlorinated polyethylene, such as identified above, from 17 to 27 percent by weight of a copolymer of ethylene vinyl acetate (EVA) and a stabilizer of up to about 3 percent by weight. Preferably, the aforementioned blend composition includes from 1 to 2 percent by weight of a calcium stearate and about 1 percent by weight of epoxidized soybean oil as stabilizers in which the soybean oil is commercially available under the trade designation Paraplex® G-60 or G-62. Normally, a stabilizer is added to thermoplastic materials in very small amounts, typically from 500 to 3000 parts per million (ppm) (0.3 percent) to prevent thermal degradation of such materials at the elevated temperatures at which the materials are extruded. It has now been discovered that a substantially greater amount of a stabilizer is needed for the extrusion of chlorinated polyethylene blends not only to prevent degradation of the blend but also to essentially prevent the generation of toxic HCl vapors during extrusion of the blend. An amount of from 1 to 3 percent by weight of one or more stabilizers have proven to be effective in the extrusion of various CPE blends.

The barrier resin layer can be a vinylidene chloride-vinyl chloride copolymer, ethylene vinyl alcohol (hydrolyzed copolymer of vinyl acetate and ethylene), other barrier resins such as those identified in U.S. Patent No. 3,549,389, for example. Such barrier resins are designed to produce a film which has excellent gas and water vapor barrier properties.

The chlorinated polyethylene (CPE) blend formulation described above has been developed to balance film properties and extrudability of the resin blend. The more CPE resin in the blend, the quieter the film but the more difficult it is to extrude. The higher the chlorine content in the blend, the more likely the resin blend will thermally degrade at extrusion temperatures. Other properties also depend on the percentage of CPE in the blend. Thus the degree of softness and comfort is increased with an increase in the percentage of CPE in the blend because the CPE resin has a relatively low modulus when compared to other resins. Further, as the chlorine level of CPE is increased, the modulus can be lowered to increase quietness and comfort. The composite films with the CPE blend of this invention are also tough (as defined by dart impact strength) even though tensile strength properties are not as high as in other films. The percentage of CPE in the blend can also affect sealability of the film as the CPE has a somewhat lower heat seal strength than that of polyethylene generally. However, the higher percentage of CPE in the blend can advantageously affect the radio frequency sealing because of its dielectric nature. The percentage of CPE in the resin blend does not significantly affect oxygen and vapor barrier properties since that is basically controlled by the barrier layer.

The dart impact strength of various monolayer and composite film laminates were tested, including 3 layered films including CPE blend skin layers of the invention. The dart impact strength tests were conducted according to ASTM D-1709 which determines the energy that causes a film to fail under specified conditions of impact of a free-falling dart. The energy is expressed in terms of the weight (mass) of the dart falling from a specified height which would result in 50 percent failure of the specimens tested. Two methods are described: Method A employs a dart with a 38 mm diameter hemispherical head dropped from a height of 0.66 m. This method is used for films whose impact resistance requires masses of about 50 g or less to about 2 kg to fracture them. Method B employs a dart with a 51 mm diameter hemispherical head dropped from a height of 1.52 m. Its range of applicability is from about 0.3 kg to 2 kg. The Standard Staircase testing method was used in which a uniform missile weight increment is employed during the test and the missile weight is decreased or increased by the uniform increment after test of each specimen, depending on the result (fail or no fail) observed for the specimen. The following results were observed as indicated in the following Table:

®Registered Trademark

**0 059 739**

TABLE I

| | Drop height (m) | Film thickness (μm) | Dart weight (gram) |
|---|---|---|---|
| 1. LDPE Film | 0.66 | 51 | 80—120 |
| | 0.66 | 76 | 120—190 |
| 2. LDPE/EVA—2 layer film | 0.66 | 51 | 150—170 |
| | 0.66 | 76 | 190—270 |
| | 0.66 | 102 | 280—290 |
| 3. LDPE/EVA/SARAN®/EVA/LDPE— 5 layer film | 0.66 | 38 | 120—130 |
| | 0.66 | 76 | 130—165 |
| 4. CPE-EVA/SARAN®/CPE-EVA— 3 layer film | 0.66  1.52 | 76 | >700 |
| CPE/EVA blend of 70 wt. percent | 0.66  1.52 | 102 | >800 |
| CPE/30 wt. percent EVA | 0.66  1.52 | 127 | >1000 |

| | Drop height (m) | CPE-LDPE blend | Film thickness (μm) | Dart weight (gram) |
|---|---|---|---|---|
| 5. CPE blends/SARAN®/ | 1.52 | 80—20 | 76 | 370 |
| CPE blends—3 layer | 1.52 | 70—30 | 76 | 320 |
| film | 1.52 | 60—40 | 76 | 250 |
| | 1.52 | 40—60 | 66 | 165 |
| | 1.52 | 20—80 | 66 | 140 |
| | 1.52 | 0—100 | 63 | 140 |

From the above Table, it should be noted that the impact strength of a monolayer film of LDPE in Experiment 1, having a thickness of 76 μm is relatively low at 120—190 grams. The impact strength of a composite film of LDPE/EVA in Experiment 2 of the same thickness of 76 μm is slightly improved at 190 to 270 grams due to the presence of the EVA layer. The 5-layered film in Experiment 3 illustrates that a layer of SARAN® reduces the impact strength of a 76 μm thick film to 130—165 grams. Substitution of the CPE-EVA blend film in Experiment 4 for the LDPE film in Experiment 3 illustrates that the impact strength of a 76 μm thickness film in Experiment 4 according to the invention is substantially improved with a dart weight of greater than 700 grams. Experiment 5 illustrates that the dart weight increases with an increase in the amount of CPE present in the blend from 140 grams (0 percent CPE) to 370 grams (80 percent CPE/20 percent LDPE).

In Experiment 2, the EVA layer had a thickness of about 10 μm. In Experiment 3, each of the EVA layers had a thickness of about 5.1 μm; the SARAN® layer had a thickness of about 6.3 μm, and the remaining thickness of the composite film being divided equally between the LDPE layers. In Experiment 4, the SARAN® layer had a thickness of about 6.3 μm with the remaining thickness of the composite films being divided equally between the CPE blend layers.

In Experiments 4 and 5, the CPE component of the blend included about 2-1/2 percent by weight of a stabilizer of which about 1-1/2 percent by weight was calcium stearate and about 1 percent by weight was epoxidized soybean oil-grade G-60.

It generally is desirable to maintain as simple a structure as possible. A three-layer CPE blend skin/polyvinylidene chloride resin core/CPE blend skin combination is contemplated as a preferred embodiment of this invention. There is ordinarily enough natural adhesion between the skin and core layer such that an intermediate glue layer is not required. However, there may be particular instances for a film having more than three layers where intermediate glue layers become desirable. For example, a five-layer composite film comprising CPE blend skin/glue layer/polyvinylidene chloride resin core/glue layer/CPE blend skin can be formed in which the two intermediate glue layers may be composed of a CPE blend which is extruded at a lower temperature than the outer skin layers of CPE blend to improve the output rate as well as the haze condition of the film. The CPE blend glue layer may also be of a different composition than the CPE blend skin layer. A hotter polymer on the skin layer provides a smoother surface to achieve improved haze condition and processability. A cooler intermediate layer protects a heat sensitive core

®Registered Trademark

layer. The intermediate CPE blend layer between the skin and core layers can also be replaced by a glue layer. As is well known in the art, various glue compositions may be used as the glue layer depending on the characteristics desired for a composite film and the film materials used. Accordingly, synthetic resinous adhesive compositions may include low, linear low, or medium density polyethylene or ethylene copolymers which comprise ethylene with at least one monoethylenically unsaturated comonomer, especially another lower olefin or a carboxylic acid or an alkyl ester of a monoethylenically unsaturated carboxylic acid. Examples of such copolymers include ethylene-propylene, ethylene acrylic acid, ethylene methyl methacrylate and ethylene vinyl acetate. Blends of one or more of the ethylenic polymer resins may also be used as the adhesive layer composition. The selection of the glue layer material is dependent on the composition of the skin and core layers.

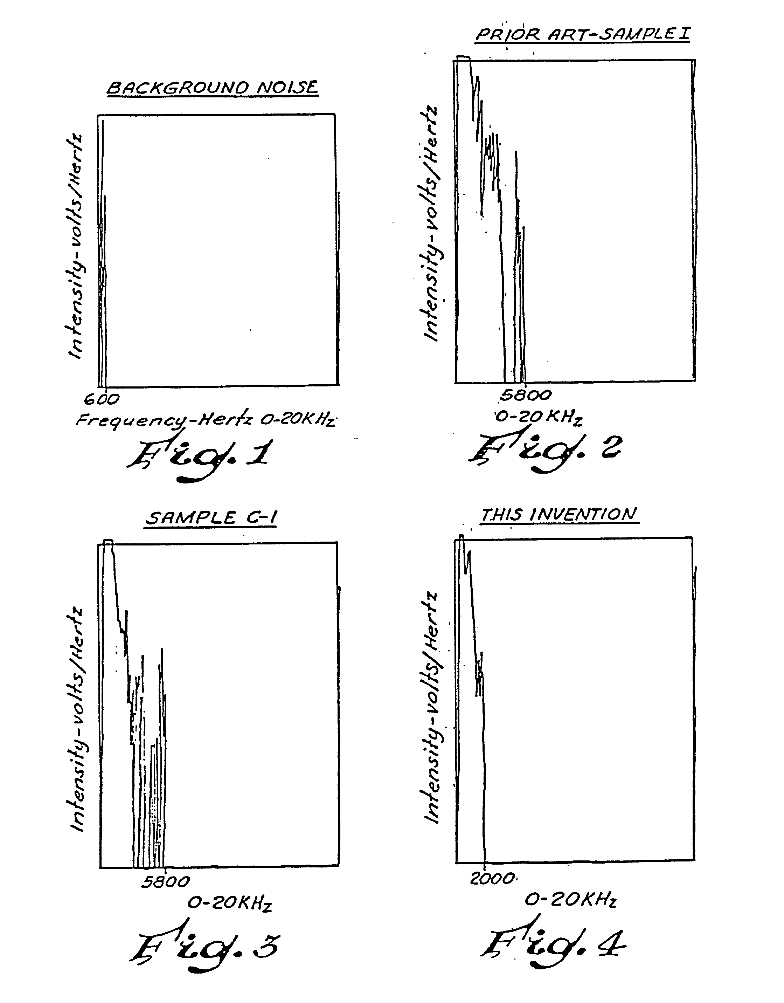

In order to determine the quietness of the composite film of the present invention with that of prior art, other films such as films used in ostomy pouches and similar containers, for example, an apparatus was constructed to flex plastic films in a controlled, repeatable manner. This apparatus utilizes an 8.5 by 8.5 centimeter sample that is fastened to a holder in such a manner that the film sample forms a cylinder. The holder is fashioned such that one end of the cylindrical film sample is held in a fixed position while the other end is rotated about the axis of the cylindrical film. Noise is generated when a wheel drive is put into contact with the rotating end of the sample holder and causes it to rotate 15.3° in one direction and 15.3° in the other direction. The frequency of this movement in opposite directions is taken at 1 cycle per second. A microphone is used to pick up the noise and the thus generated noise is analyzed to determine its frequency in a Harmonic Spectrum analyzer. The sound created by a sample in the above manner was measured by placing the microphone in the fixed end of the sample holder thereby generating an electrical signal that is processed or recorded as desired, as typified in Figures 2 to 4 of the drawing. To determine decibel readings the microphone of the spectrum analyzer was replaced with a microphone of an audiometer to pick up the decibel readings. By this technique a direct reading of the sound level over the entire hearing range is available as a single figure expressed in dBA. The dBA unit was selected to report results as it is an accepted decibel noise unit used by most authorities.

Tests were run of film samples having various quantities of chlorinated polyethylene ("CPE") resin blended with either low density polyethylene ("LDPE") or linear low density polyethylene ("LLDPE"). The noise levels (or degree of quietness) were compared against background room noise and the noise generated by films currently commercially available, including films having a relatively high degree of quietness in motion. It was initially thought that a comparison of decibel (dBA) readings would define the apparent and perceived improvement in quietness achieved by films of the present invention over films of the prior art. Surprisingly, this did not occur in every instance. Because of this, the frequency value of the various film samples was also checked. This did consistently confirm the apparent and perceived quietness improvement achieved by films of the present invention. Table II summarizes the general results of the findings of the tests.

6

# 0 059 739

TABLE II

| Sample identification | Material* composition | Total film thickness** in µm | dBA Reading | Frequency** reading in Hertz units |
|---|---|---|---|---|
| Background noise | — | — | 56 | 600 |
| Prior art Sample I | EVA/PVDC/EVA | 76 | 77 | 5,800 |
| Prior art Sample II | Monolayer PVC | 218 | 59 | 750 |
| Prior art Sample III | PE/EVA/PVDC/EVA/PE | 71 | 86 | 11,800 |
| Sample A-1 | 0% CPE, 100% LDPE | 66 | 87 | 10,000 |
| Sample A-2 | 0% CPE, 100% LLDPE | 71 | 83 | 6,500 |
| Sample B-1 | 17.5% CPE, 80% LDPE | 69 | 83 | 5,000 |
| Sample B-2 | 17.5% CPE, 80% LLDPE | 76 | 79 | 6,700 |
| Sample C-1 | 37.5% CPE, 60% LDPE | 71 | 78 | 5,800 |
| Sample C-2 | 37.5% CPE, 60% LLDPE | 76 | 75 | 7,000 |
| Sample D-1 | 57.5% CPE, 40% LDPE | 74 | 77 | 3,800 |
| Sample D-2 | 57.5% CPE, 40% LLDPE | 79 | 75 | 3,000 |
| Sample E-1 | 67.5% CPE, 30% LDPE | 76 | 72 | 2,000 |
| Sample E-2 | 67.5% CPE, 30% LLDPE | 71 | 74 | 2,600 |
| Sample F-1 | 77.5%·CPE, 20% LDPE | 84 | 75 | 3,500 |
| Sample F-2 | 77.5% CPE, 20% LLDPE | 84 | 74 | 3,000 |
| Sample G-1 | 70.0% CPE, 27-1/2% EVA | 76 | 72.5 | |
| Sample H | 97.5% CPE, 0% LDPE or LLDPE | 69 | 57 | 800 |

\* PVC=Polyvinyl chloride; CPE=chlorinated polyethylene; LDPE=low density polyethylene; LLDPE=linear low density polyethylene; EVA=ethylene vinyl acetate copolymer resin such as Elvax brand resin produced by duPont Chemical Company; PVDC=polyvinylidene copolymer such as SARAN® resin produced by The Dow Chemical Company. Approximate compositions where known. Samples B-1 through Sample H include about 2.5% stabilizers in addition to the CPE blend. Each of the films of Samples B-1 through G are multilayered films having a PVDC core layer, the materials of the skin layers being designated in the Table. Sample H is a monolayer film.

\*\* Approximate rounded off readings.

As can be determined from Table II, there are varying degrees of difference in the recorded decibel noise level of the films. The group of films of Prior Art Samples I and III and Samples A-1 through G, are all in the 72 to 87 dBA range, which provides some indication of relative quietness. Yet a perceptible difference in quietness is realized by those handling films of a similar dBA reading, such as Samples D-1 through G-1 as compared with Prior Art Sample I and Samples C-1 and C-2 of this group. This perceived difference registers in the frequency readings where Samples D-1 through G-1 all show frequency readings of less than 4000 Hertz while the remainder show frequency readings considerably in excess of 4000 Hertz. These frequency readings are all relative to the background noise level of about 600 Hertz.

To more clearly understand the meaning and significance of the improvements of this invention, reference can be made to the Drawing. Figure 1 illustrates graphically by the jagged line in the chart the intensity and frequency of the background noise found in the room where the testing occurred. The frequency level of the background noise in this instance was about 600 Hertz. When typical prior art film and other film samples were tested, Samples I and C-1, graphically illustrated in Figures 2 and 3,

® Registered Trademark.

respectively, demonstrated a frequency reading of about 5800 Hertz, considerably above the room background noise level of 600 Hertz, and clearly audible to an observer in the room. In comparison, the film sample of this invention had a frequency reading of less than 4000 Hertz, and as low as about 2000 Hertz for Sample E-1, which is graphically illustrated in Figure 4. This graphic representation of Figure 4 is much closer to that of the background noise of Figure 1 than to that of Figures 2 and 3. Samples E-1 and E-2 are preferred because of their low noise levels, but Samples D-1, D-2, F-1, and F-2 are suitable quiet films made in accordance with the discovery of this invention. The films of the present invention are extremely quiet, can be readily extruded and either heat or dielectrically sealed, and have excellent water vapor and odor barrier properties.

The films of Prior Art Sample II and Sample H exhibit quietness levels below that of the films of this invention, both in the dBA and frequency readings. However, prior Art Sample II is a polyvinyl chloride monolayer film and is about three times as thick as the film of this invention. This film, while very soft and quiet, has extrememly poor water vapor barrier properties, per thickness unit, when compared with other films such as polyethylene or polyvinylidene chloride films. It, therefore, is necessary to make an extremely thick film to have anything approaching adequate vapor barrier properties using polyvinyl chloride resin.

Sample H, while quiet, comprises a heavy concentration of the relatively expensive chlorinated polyethylene resin. This results in higher costs when compared with the other film samples using blends of polyethylene in combination with chlorinated polyethylene. Also, the heavier the concentration of chlorinated polyethylene, the more difficult it is to manufacture the film, as discussed hereinbefore.

Various properties, including the ultimate tensile strength and heat seal properties of a 3-layer composite film of the invention was compared against a prior art sample as illustrated in Table III. The ultimate tensile properties of the thermoplastic film were tested in accordance with ASTM D-882 Method A which is a static weighing-constant-Rate-of-Grip Separation Test. This method employs a constant rate of separation of the grips holding the ends of the test specimen. Samples 1, 2 and 3 employed a 3-layered structure in which the skin layers were each composed of a blend of 70 percent by weight CPE; 27 percent by weight EVA and 3 percent by weight of a stabilizer mixture. Sample 4 was a prior art sample of a 5-layer structure of PE/EVA/PVDC/EVA/PE.

TABLE III

| Film sample | Gauge (µm) | Noise level (dBA) [Hertz] | Ultimate tensile psi (kg/cm²) | 0.66 m Dart drop (gram) | Heat seal 360°F (177°C); 30 psi (2.1 kg/cm²) 1 sec. dwell lb/in (kg/cm) |
|---|---|---|---|---|---|
| 1. | 76 | 71.0 [1800 bg-910] | MD 3535(248) TD 978(69) | >700 | MD 3.7(0.66) TD 2.4(0.43) |
| 2. | 102 | 73.5 | MD 3276(229) TD 1122(79) | >800 | MD 3.7(0.66) TD 2.8(0.50) |
| 3. | 127 | 75.2 | MD 2856(200) TD 1046(73) | >1000 | MD 3.9(0.70) TD 3.3(0.59) |
| 4. | 71 | 86 | MD 2697(189) TD 2163(152) | 125 | MD 6.3(1.13) TD 4.9(0.88) |

MD—Machine Direction
TD—Transverse Direction
bg—Background Noise.

The noise level of the composite films in Samples 1, 2 and 3 showed an improvement in all thicknesses over the noise level of Sample 4 having a composite film thickness of 71 µm. The frequency reading for film Sample 1 was found to be well within the desired range at less than 1800 Hertz with a background noise of 910 Hertz. The ultimate tensile strength in the machine direction compared favorably with the prior art sample while the dart drop test showed a substantial improvement over the prior art sample. Similarly, the heat seal test compared favorably with that of Sample 4.

Various properties, including the ultimate tensile strength, heat seal properties and dart impact strength were tested on a 3-layer film comprising outer skin layers of a blend of chlorinated polyethylene and linear low density polyethylene and a core layer of SARAN®. The polymeric blend varied in proportion from 0 percent CPE to a maximum of 80 percent CPE. The CPE component included 1.5 parts of calcium stearate and 1.0 part of PARAPLEX® G-60 as stabilizers per 100 parts of CPE. An antiblocking agent ($SiO_2$) was added in an amount of 3.5 parts per 100 parts of CPE. The following results were observed as indicated in Table IV.

TABLE IV

| Film description | Thickness in μm (dBA)[1] [Hertz][2] | Ultimate tensile PSI (kg/cm²) | Heat seal 350°F (177°C) 30 psi (2.1 kg/cm²) 3 sec. dwell lb/in (kg/cm) | Dart weight[3] (grams) |
|---|---|---|---|---|
| 80% CPE 20% LLDPE | 76 (74.0) [3860] | MD 1920(134) TD 850(59) | MD 4.6(0.82) TD 5.5(0.98) | 7400 |
| 70% CPE 30% LLDPE | 76 (73.5) [2860] | MD 2260(158) TD 1220(85) | MD 5.5(0.98) TD 5.2(0.93) | 7400 |
| 60% CPE 40% LLDPE | 71 (75.0) [3260] | MD 2300(161) TD 1450(101) | MD 5.1(0.91) TD 5.4(0.97) | 379 |
| 40% CPE 60% LLDPE | 71 (75.0) [5430] | MD 2350(164) TD 1800(126) | MD 2.0(0.36) TD 2.2(0.39) | 307 |
| 20% CPE 80% LLDPE | 66 (78.8) [7660] | MD 2486(174) TD 2190(153) | MD 2.3(0.41) TD 1.9(0.34) | 223 |
| 0% CPE 100% LLDPE | 63 (83.0) [6710] | MD 2810(197) TD 2520(176) | MD 2.0(0.36) TD 2.2(0.39) | 170 |

[1] Background noise (56.0)—dBA
[2] Background noise [570]—Hertz
[3] Height of drop 66 cm
MD—Machine Direction
TD—Transverse Direction Multi-layered film (as in Table V but using LLDPE)

®Registered Trademark

**0 059 739**

TABLE V

| Film description | Thickness in μm (dBA)[1] [Hertz] | Ultimate tensile psi (kg/cm²) | Heat seal 350°F (177°C) 30 psi (2.1 kg/cm²) 3 sec. dwell lb/in (kg/cm) | Dart weight[3] (grams) |
|---|---|---|---|---|
| 80% CPE 20% LDPE | 76 (75.0) [3660] | MD 2177(152) TD 1076(75) | MD 4.45(0.797) TD 4.25(0.76) | 373 |
| 70% CPE 30% LDPE | 76 (72.0) [2290] | MD 2271(159) TD 1170(82) | MD 4.5(0.8) TD 4.5(0.8) | 322 |
| 60% CPE 40% LDPE | 76 (77.0) [3740] | MD 2494(175) TD 1188(83) | MD 3.7(0.66) TD 3.1(0.55) | 253 |
| 40% CPE 60% LDPE | 66 (77.8) [5860] | MD 2566(180) TD 1304(91) | MD 1.9(0.34) TD 1.5(0.27) | 165 |
| 20% CPE 80% LDPE | 66 (82.5) [5170] | MD 2808(197) TD 1835(128) | MD 1.5(0.27) TD 1.05(0.19) | 144 |
| 0% CPE | 61 (87.0) [12400] | MD 3587(251) TD 1941(136) | MD 2.2(0.39) TD 1.1(0.2) | 136 |

All are 3 layer structures CPE+LDPE/SARAN®/CPE LDPE
[1] Background noise (56.0)—dBA
[2] Background noise [570]—Hertz
[3] Height of drop 66 cm
MD—Machine Direction
TD—Transverse Direction

Although the ultimate tensile strength of the film decreased with an increase in CPE in the blend, the tensile strength for a blend of 80 percent CPE-20 percent LLDPE was found to be fully deficient. Heat seal strength and dart impact strength both showed substantial increases in strength with an increase in CPE in the blend. An improvement was also observed in the quietness of the film materials as the level of CPE in the blend was increased.

The composite multilayer film of the present invention is a superior product capable of providing adequate service when compared to existing products yet maintaining a high degree of quietness so that applications of the product in the form of containers, such as ostomy bags where rustle-free characteristics are of major significance, can be achieved. The products to be made from the film may not only reside in film pouches for surgical or other medical use, but may find application in many other products where quietness is an important factor, such as in clothing, diapers or in wrappings and other packaging uses.

**Claims**

1. A thermoplastic multilayer composite film having improved dart impact strength comprising at least one layer (a) formed from a composition of a blend of polymeric resins wherein a first component of said blend comprises from 40 to 95 weight percent of a chlorinated poly olefin having from 25 to 50 weight percent chemically combined chlorine, and wherein a second component of the blend comprises from 2 to 57 weight percent of at lesat one olefinic polymer or copolymer, and from 1 to 3 weight percent of a stabilizing composition to prevent degradation of the chlorinated poly olefin at extrusion temperatures for forming the film, and at least one layer (b) formed from another polymeric material adhered to at least one surface of said layer (a).

2. The composite film according to claim 1 wherein the first component of the blend for layer (a) comprises from 55 to 80 weight percent chlorinated polyethylene having from 30 to 42 weight percent chemically combined chlorine and the second component comprises from 17 to 42 weight percent of at least one olefinic polymer or copolymer.

® Registered Trademark.

10

3. The composite film of claim 1 or 2, wherein the second component of the blend for layer (a) is selected from a low, linear low, or medium density polyethylene, and a copolymer of ethylene and at least one ethylenically unsaturated comonomer.

4. The composite film of claim 1, 2 or 3 having a noise level of less than 4000 Hertz when measured against a background noise of about 600 Hertz.

5. The composite film of any of the preceding claims wherein the layer (a) of the film has a thickness from 63 to 152 μm.

6. The composite film of any of the preceding claims wherein the stabilizing composition of the blend for layer (a) comprises 1 to 2 percent by weight calcium stearate and 1 percent by weight of epoxidized soybean oil.

7. The composite film of any of the preceding claims wherein layer (b) is a vapor barrier layer and the total thickness of the composite film is from 25.4 to 254 μm.

8. The composite film of any of the preceding claims wherein the vapor barrier layer (b) is selected from a vinylidene chloride-vinyl chloride copolymer.

9. The composite film of any of the preceding claims wherein a layer (a) is adhered to opposite sides of layer (b).

10. The composite film of any of the preceding claims including at least one synthetic resinous glue layer positioned between layer (a) and layer (b).

11. The composite film of claim 10 including at least one synthetic resinous glue layer positioned on at least one surface of layer (a) or on layer (b).

12. The composite film of any of the preceding claims wherein the film is laminated or coextruded onto a substrate.

13. A wrapping film comprising a multilayered composite film of any of claims 1 to 12.

14. A medical pouch comprising a multilayered composite film of any of claims 1 to 12.

**Patentansprüche**

1. Thermoplastische Mehrschichtfolie mit verbesserter Schlagzähigkeit nach der Kugelfallmethode, die mindestens eine Schicht (a) gebildet aus einer Zusammensetzung von polymeren Harzen mit einer ersten Komponente von 40 bis 95 Gew.% eines chlorierten Polyolefins mit 25 bis 50 Gew.% chemisch gebundenem Chlor und einer zweiten Komponente von 2 bis 57 Gew.% eines anderen olefinischen Polymeren oder Copolymeren und von 1 bis 3 Gew.% einer Stabilisatorzusammensetzung, um die Zersetzung des chlorierten Polyolefins bei den Extrusionstemperaturen beim Ausbilden der Folie zu verhindern und mindestens eine Schicht (b) aus einem anderen polymeren Material aufweist, die an mindestens einer Oberfläche der Schicht (a) haftet.

2. Zusammensetzung nach Anspruch 1, dadurch gekennzeichnet, daß die Mischung der Schicht (a) als erste Komponente von 55 bis 80 Gew.% chloriertes Polyethylen mit von 30 bis 42 Gew.% chemisch gebundenem Chlor und als zweite Komponente von 17 bis 42 Gew.% mindestens eines olefinischen Polymer oder Co-Polymer enthält.

3. Mehrschichtfolie nach Ansprüchen 1 oder 2, dadurch gekennzeichnet, daß die zweite Komponente der Mischung der Schicht (a) ausgewählt ist aus einem niedrig-, linear-niedrig- oder mitteldichtem Polyethylen und einem Copolymer von Ethylen und mindestens einem ethylenisch ungesättigten Comonomeren.

4. Mehrschichtfolie nach Ansprüchen 1, 2 oder 3, dadurch gekennzeichnet, daß sie einen Lärmpegel von weniger als 4000 Hz gemessen gegen einen Untergrundlärm von etwa 600 Hz aufweist.

5. Mehrschichtfolie nach jedem der vorstehenden Ansprüche, dadurch gekennzeichnet, daß die Schicht (a) der Folie eine Dicke von 63 bis 152 μm aufweist.

6. Mehrschichtfolie nach jedem der vorstehenden Ansprüche, dadurch gekennzeichnet, daß die Stabilisierungszusammensetzung der Mischung der Schicht (a) 1 bis 2 Gew.% Calziumstearat und 1 Gew.% epoxidiertes Sojabohnenöl enthält.

7. Zusammensetzung nach jedem der vorstehenden Ansprüche, dadurch gekennzeichnet, daß die Schicht (b) eine dampfsperrende Schicht ist und die Gesamtdicke der Mehrschichtfolie von 25,4 bis 254 μm beträgt.

8. Mehrschichtfolie nach jedem der vorstehenden Ansprüche, dadurch gekennzeichnet, daß die dampfsperrende Schicht ein Vinylidenchlorid-Vinylchlorid-Copolymer ist.

9. Mehrschichtfolie nach jedem der vorstehenden Ansprüche, dadurch gekennzeichnet, daß die Schicht (a) auf beiden gegenüberliegenden Seiten der Schicht (b) angeordnet ist.

10. Mehrschichtfolie nach jedem der vrostehenden Ansprüche, dadurch gekennzeichnet, daß mindestens eine synthetische Harzleimschicht zwischen der Schicht (a) und der Schicht (b) angeordnet ist.

11. Mehrschichtfolie nach Anspruch 10, dadurch gekennzeichnet, daß mindestens eine synthetische Harzleimschicht auf mindestens einer Oberfläche der Schicht (a) oder der Schicht (b) angeordnet ist.

12. Mehrschichtfolie nach jedem der vorstehenden Ansprüche, dadurch gekennzeichnet, daß die Folie auf ein Substrat laminiert oder co-extrudiert ist.

11

13. Verpackungsfolie, dadurch gekennzeichnet, daß sie eine Mehrschichtfolie nach jedem der Ansprüche 1 bis 12 enthält.

14. Medizinischer Beutel, dadurch gekennzeichnet, daß er eine Mehrschichtfolie nach jedem der Ansprüche 1 bis 12 enthält.

**Revendications**

1. Film composite multicouche thermoplastique ayant une résistance aux chocs améliorée comprenant au moins une couche (a) formée à partir d'une composition d'un mélange de résines polymères, dans lequel un premier constituant de ce mélange comprend de 40 à 95% en poids d'une polyoléfine chlorée ayant de 25 à 50% en poids de chlore chimiquement combiné et dans lequel un second constituant du mélange comprend de 2 à 57% en poids d'au moins un polymère ou copolymère oléfinique, et de 1 à 3% en poids d'une composition stabilisante pour empêcher la dégradation de la polyoléfine chlorée aux températures d'extrusion pour former le film, et au moins une couche (b) formée à partir d'une autre matière polymère collée à au moins une face de cette couche (a).

2. Filme composite selon la revendication 1, dans lequel le premier constituant du mélange pour la couche (a) comprend de 55 à 80% en poids de polyéthylène chloré ayant de 30 à 42% en poids de chlore combiné chiimiquement, et le second constituant comprend de 17 à 42% en poids d'au moins un polymère ou copolymère oléfinique.

3. Film composite selon l'une des revendications 1 ou 2, dans lequel le second constituant du mélange pour la couche (a) est choisi parmi un polyéthylène basse densité, basse densité linéaire, ou moyenne densité, et un copolymère de l'éthylène et d'au moins un comonomère à insaturation éthylénique.

4. Film composite selon l'une des revendications 1, 2 ou 3, ayant un niveau de bruit inférieur à 4000 Hz lorsqu'il est mesuré contre un bruit de fond d'environ 600 Hz.

5. Film composite selon l'une des revendications précédentes, dans lequel la couche (a) du film a une épaisseur de 63 à 152 μm.

6. Film composite selon l'une des revendications précédentes, dans lequel la composition stabilisante du mélange pour la couche (a) comprend 1 à 2% en poids de stéarate de calcium et 1% en poids d'huile de soja époxydée.

7. Film composite selon l'une des revendications précédentes, dans lequel la couche (b) est une couche barrière de vapeur et l'épaisseur totale du film composite est de 25,4 à 254 μm.

8. Film composite selon l'une des revendications précédentes, dans lequel la couche barrière de vapeur (b) est choisie parmi un copolymère de chlorure de vinylidène-chlorure de vinyle.

9. Film composite selon l'une des revendications précédentes, dans lequel une couche (a) est collée sur des côtés opposés de la couche (b).

10. Film composite selon l'une des revendications précédentes, contenant au moins une couche de colle résineuse synthétique disposée entre la couche (a) et la couche (b).

11. Film composite selon la revendication 10, contenant au moins une couche de colle résineuse synthétique disposée sur au moins une face de la couche (a) ou sur la couche (b).

12. Film composite selon l'une des revendications précédentes, dans lequel le film est stratifié ou co-extrudé sur un substrat.

13. Film d'enveloppement comprenant un film composite multicouche selon l'une des revendications 1 à 12.

14. Poche médicale comprenant un film composite multicouche selon l'une des revendications 1 à 12.

BACKGROUND NOISE

Fig. 1

PRIOR ART-SAMPLE I

Fig. 2

SAMPLE C-I

Fig. 3

THIS INVENTION

Fig. 4